# EUROPEAN PATENT APPLICATION

(11) **EP 2 742 955 A1**
(43) Date of publication of application: **18.06.2014**
(21) Application number: 12306573.2
(22) Date of filing: 12.12.2012
(51) Int. Cl.: A61K 47/48, A61K 31/7024, A61P 7/02

(54) **Nanoparticles based on bioconjugate of GAG**

(71) Applicant: Centre National de la Recherche Scientifique, 75794 Paris Cedex 16 (FR); UNIVERSITE PARIS-SUD 11, 91400 Orsay (FR)
(72) Inventor: Ralay-Ranaivo, Bettina, 92190 Meudon (FR); Couvreur, Patrick, 91140 Villebon sur Yvette (FR); Desmaele, Didier, 94260 Fresnes (FR); Bianchini, Elsa, 91140 Villebon sur Yvette (FR); Borgel, Delphine, 75016 Paris (FR); Gref, Ruxandra, 91370 Verrieres le Buisson (FR)
(74) Representative: Nony

(57) **Abstract**

The present invention relates to nanoparticles comprising at least one negatively charged glycosaminoglycan-type macromolecule or a derivative thereof, non-covalently coupled to at least one molecule of a cationic hydrocarbon-based radical of squalene nature represented by the formula (I) which follows: 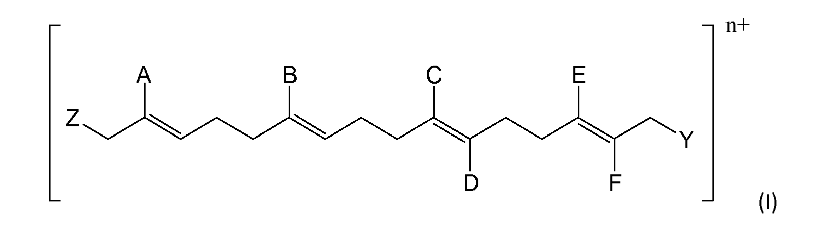 wherein:
A, B, C, D, E and F are a hydrogen atom or a methyl group; Z and Y represent a radical 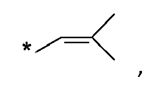 an ethyltrialkylammonium radical, an ethylguanidium radical or a 1-[ethylideneamino]guanidinium radical with the proviso that at least one of Z and Y is different from 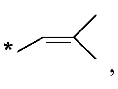 the symbol * showing the position of the attachment of the radical to the structure; and n is equal to 1 or 2.

The present invention also relates to a method for preparing said nanoparticles, and to a lyophilisate, a solid dosage form and pharmaceutical or dermatological compositions comprising said nanoparticles. Said nanoparticles are useful as medicines and more particularly as anticoagulant agents.

## Description

The present invention relates to original nanoparticles useful for administration and in particular by oral route, of active molecules having a very low bioavailability, like in particular fondaparinux (Fpx) useful for treatment of thromboembolic disorders.

Thromboembolic disorders are among the major causes of morbidity and mortality and anticoagulation remains the therapy of choice for prevention and treatment of these venous and arterial thromboembolic disorders.

Currently, heparin (unfractionated heparin (UFH) and low molecular weight heparin (LMWH)), fondaparinux (Fpx) and vitamin K antagonists (VKA) are the primary treatments for venous thromboembolic.

Heparin, commonly known as UFH is a highly sulfated polysaccharide that displays anticoagulant activity by the inhibition of the coagulation factors, factor Xa (FXa) and thrombin through binding with the plasma protein inhibitor of coagulation, antithrombin (AT). However, its low bioavailability and its high interindividual variability led to the development of LMWH obtained by fractionation of heparin.

Compared with UFH, LMWH exhibits greater bioavailability after subcutaneous (SC) injection, has a longer half-life and produces a more predictable anticoagulant response without routine monitoring. However, LMWH still retains the potential to cause Heparin-induced thrombocytopenia (HIT).

In 2001, Fpx, a synthetic and structural analog of the AT-binding pentasaccharide domain of heparin was developed as a new antithrombotic drug. Fpx is a synthetic α-methyl pentasaccharide derivative, which has a sequence derived from the AT binding region of heparin. It has the lowest molecular weight (1728 Da) to enable a biological activity among heparins.

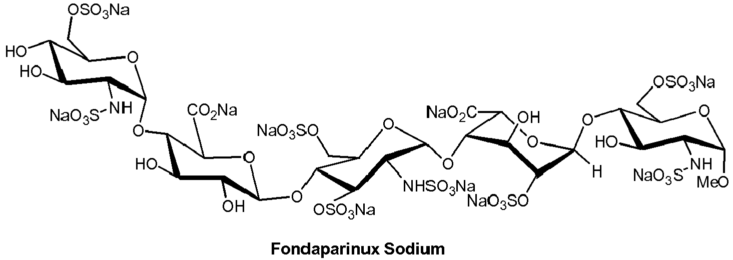

Compared to UFH and LMWH, Fpx has selective anti-Xa activity and longer half-life. Accordingly, since its introduction on the market in 2002, fondaparinux became an anticoagulant of choice in the treatment of short- and medium-term thromboembolic disease with the unfractionated heparin (UFH) and low molecular weight heparin (LMWH). However, although its molecular weight (1728 Da) is much lower than the one of LMWH, Fpx remains a hydrophilic polyanionic macromolecule showing a very low bioavailability by oral route. This low oral absorption is the result of: i) a poor transport through the intestinal epithelial barrier; ii) a pronounced instability in acidic pH conditions in the stomach; and iii) a fast enzymatic degradation. Due to these drawbacks, Fpx is only administrated via subcutaneous route.

It is not questionable that oral delivery is the preferred route of administration and since 2009, the development of an oral form of Fpx started to attract interest.

In this respect, different approaches such as the use of permeation enhancers, the conjugation with lipids and the development of carriers and solid dosage forms loaded with such active anionic polysaccharides have already been proposed. These prior approaches showed effectively significant enhancements in the bioavailability of the macromolecular drugs but unfortunately with some effectiveness depending on the system.

Accordingly, there is still a need for efficient oral delivery forms for Fpx and more generally for active compounds exhibiting the same problems i.e. a limited permeability in the gastrointestinal tract, a rapid enzymatic and metabolic degradation, and/or a chemical and biological instability.

More generally, these compounds presenting these problems are Glycosaminoglycan-type (GAG) macromolecules. Glycosaminoglycan-type macromolecules are highly sulphated not branched polysaccharides that comprise repeating disaccharides of an uronic acid (iduronic or glucuronic) linked to an aminosugar (glucosamine or galactosamine). Representatives of (GAG) are heparin and its derivatives, LMWH (Low Molecular Weight Heparins), pentasaccharides (like fondaparinux), ULMWH (Ultra Low Molecular Weight Heparins), Chondroitin sulphate, Dermatan Sulphate, etc.

Thus, one of the aspects of the instant invention is to propose a new way for formulating glycosaminoglycan-type macromolecules under efficient oral delivery forms.

As hereafter detailed, the inventors of the present invention discovered that it is possible to formulate such active compounds in the form of solid nanoparticles of small sizes, in particular compatible with oral administration, with the proviso that these active compounds are non-covalently coupled to at least one cationic hydrocarbon-based radical of squalene nature.

Thus, according to a first aspect, the present invention relates to nanoparticles comprising at least one negatively charged glycosaminoglycan-type (GAG) macromolecule or a derivative thereof, non-covalently coupled to at least one molecule of a cationic hydrocarbon-based radical of squalene nature represented by the formula (I) which follows: 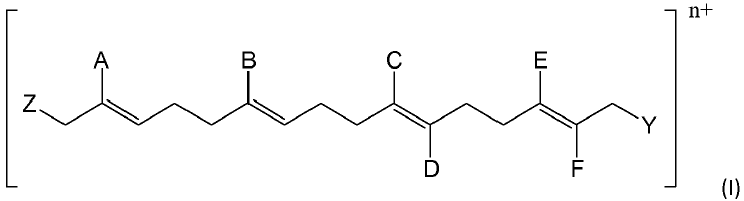 wherein:
- A, B, C, D, E and F are, independently ones from the others, a hydrogen atom or a methyl group,
- Z and Y, independently one from the other, represent a radical 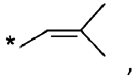 an ethyltrialkylammonium radical, an ethylguanidium radical (also named ethylguanidinium radical) or a 1-[ethylideneamino]guanidinium radical with the proviso that at least one of Z and Y is different from 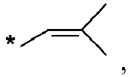 the symbol * showing the position of the attachment of the radical to the structure, and
- n is equal to 1 or 2.

According to a preferred embodiment, n is equal to 1.

According to a preferred embodiment, at least one Y represents one ethyltrialkylammonium radical and n is equal to 1.

According to another embodiment, Y and Z represent one ethyltrialkylammonium radical and n is equal to 2.

Advantageously, the mean size of these nanoparticles ranges from 50 to 800 nm, in particular from 75 to 500 nm, and more preferably from 100 nm to 200 nm.

The squalene is a natural lipid precursor of cholesterol in sterol biosynthesis. More recently, squalene derivatives have been covalently coupled to a series of active molecules (for example to gemcitabine (WO2006/090029), statine (EP 2 355 801) or β-lactame (EP 2 355 800)). Remarkably, the so formed squalene-bioconjugates spontaneously self-organized in water as nanoparticles (NPs) of around 200 nm. For example, in the case of gemcitabine, it has been shown that the NPs made of squalenoyl gemcitabine bioconjugates have dramatically increased anticancer activity compared to free gemcitabine (WO2006/090029). However, this ability to form nanoparticles is only shown when such a hydrocarbon-based radical is covalently coupled to the considered active molecules like for example gemcitabine.

The inventors have now developed a new nanoparticulate system based on the formation of ion pairs between cationic molecules of squalene or derivatives thereof and negatively charged active molecule without the need of covalent coupling. Unexpectedly, they have noticed the ability of positive charged squalene derivatives of formula (I) to self-organize in nanoparticles when they are coupled through ionic interaction with negative charged macromolecules like GAG.

This ability has been more particularly checked in the experimental part with Fpx. Thus, ionic (i.e. non-covalent) squalenoylated bioconjugates, Fpx and the considered positive charged squalene derivative are self-assembled in water. The driving forces are very likely electrostatic interactions as well as hydrophobic interactions of the considered positive charged squalene derivative which lead to the formation of monodispersed nanoparticles.

As indicated above, the formulation of the therapeutic active compounds under consideration according to the present invention, in the form of nanoparticles in accordance with the present invention, constitutes an advantageous alternative with regard to the formulations that already exist, in several respects.

First of all, the nanoparticulate form of GAG advantageously makes it possible to overcome the natural limited permeability of these GAG in the gastrointestinal tract.

Furthermore, the nanoparticulate form of GAG according to the invention has no significant impact on the pharmacokinetic of the drug after oral administration.

At last, the approach considered according to the invention allows to avoid: i) fastidious synthesis; ii) the risk to lose activity of the drug by chemical modification; and iii) the necessity to break the covalent bonds between the active compounds and the squalene derivatives in order to release the active compounds.

The present invention also relates to a method for preparing said nanoparticles, comprising at least the steps consisting of:
- providing a solution of a cationic compound of formula (I) as defined previously in an organic water-soluble medium,
- pouring said solution in an aqueous phase containing at least one molecule of negatively charged GAG macromolecule, the concentration of compound of formula (I) and of GAG macromolecule being adjusted to get an ionic bioconjugate which, under stirring, instantaneously forms the expected nanoparticles in suspension in said aqueous phase, and, where appropriate, the isolation of said nanoparticles.

Advantageously, said method may also comprise a subsequent lyophilization step (also named freeze-drying step), particularly suitable for being able to formulate a solid form.

Thus, the present invention also relates to a lyophilisate comprising at least the nanoparticles as described above.

According to a preferred embodiment, this lyophilisate further comprises at least a cryprotectant, in particular trehalose, glycerol and glucose, and more preferably trehalose.

According to another subject, the present invention is directed toward nanoparticles as defined above, optionally in the form of a lyophilisate as defined above, for preparing a pharmaceutical composition in particular applicable to mucosas and in particular to oropharyngeal mucosa, gastrointestinal mucosa, pulmonary mucosa, nasal mucosa and vaginal mucosa.

The present invention is also directed to solid form dosage intended for oral administration containing at least nanoparticles according to the invention, optionally in the form of a lyophilisate. This solid form dosage may advantageously be a delayed release solid form dosage like for example enteric coated tablets or capsules and which surface coating provide the delayed release.

Naturally, the claimed nanoparticles are also convenient for an administration other than oral and particular by subcutaneous route, or also topical route.

At last, the nanoparticles according to the present invention are particularly interesting with respect to a highly improved skin penetration of GAG formulated with cationic squalene derivative of formula (I) since squalene is a natural compound present on skin. It is known that GAG are essential in the anti-ageing process as they keep moisture in the skin.

Thus, the present invention concerns any pharmaceutical or dermatological composition, in particular a medicament, comprising at least nanoparticles, optionally in the form of a lyophilisate, and/or capsules (also named hereinafter nanocapsules) as described above, in combination with at least one acceptable pharmaceutical vehicle.

The present invention is also directed toward said composition for use thereof as a medicament for the treatment and/or prevention of the above-mentioned diseases and/or conditions and in particular thromboembolic diseases and in particular as anticoagulant.

Thus, the present invention also relates to nanoparticles according to the invention for use as medicines and more particularly for use as anticoagulant agents for the treatment and/or prevention of thromboembolic diseases and cardiovascular diseases such as thrombophlebitis, venous thrombosis, phlebothrombosis, deep vein thrombosis, portal vein thrombosis, renal vein thrombosis, jugular vein thrombosis, Budd-Chiari syndrome, Paget-Schroetter disease, cerebral venous sinus thrombosis, pulmonary embolism, arterial thrombosis, stroke, myocardial infarction, hepatic artery thrombosis, arterial embolus.

### NANOPARTICLES OF THE INVENTION

As stated previously, the inventors discovered that when negative charges of glycosaminoglycan-type (GAG) macromolecules as for instance Fpx, are in part or totally neutralized with cationic charges of a cationic hydrocarbon-based radical of squalene nature of formula (I), the so-formed ionic bioconjugate may self-organize in nanoparticles in suspension in an aqueous mixture wherein the active material is entrapped with very high loading.

For the glycosaminoglycan-type (GAG) macromolecules, the anionic charges are their sulfate groups and/or carboxyl groups.

For the squalene compound of formula (I), the cationic charges are its ethyltrialkylammonium, 1-[ethylideneamino] guanidinium and/or ethylguanidinium groups. The ethyltrialkylammonium, 1-[ethylideneamino] guanidinium and/or ethylguanidinium groups of the squalene compound of formula (I) allow the formation of electrostatic interaction with the sulfate groups and/or carboxylic groups of the GAG and these binding promote from one hand the entrapping of GAG with high efficiency and from other hand an ability to the so-formed ionic bioconjugate to self-organize under the form of nanoparticles in an aqueous medium.

The so-achieved nanoparticles, wherein the interaction between the molecule(s) of cationic squalene derivatives and a negatively charged glycosaminoglycan-type (GAG) macromolecule is the result of ion pair formation between both molecules, are particularly interesting, with respect to:
- their simplicity of preparation without need of heating step which could inactivate or degrade the glycosaminoglycan-type macromolecule, and consequently hamper their scale up for industrial purposes,
- their small size,
- their high level of protection for the glycosaminoglycan from enzymatic degradation in the gastrointestinal tract,
- their high loading in glycosaminoglycan-type macromolecule at least 39 wt % and until 80 wt % encapsulation efficiency,
- their ionic interactions which do not prevent their disruption to release the encapsulated GAG, and
- their non-alteration of the native activity of glycosaminoglycan-type macromolecule
- at last, an highly improved skin penetration of GAG formulated with cationic squalene derivative of formula (I) since squalene is a natural compound present on skin. It is known that GAG are essential in the anti-ageing process as they keep moisture in the skin.

As noted by the inventors, the bioconjugate formed by interaction of ion pair(s) between cationic squalene derivative of formula (I) as defined above and the negatively charged molecule of glycosaminoglycan spontaneously displays, when it is brought into contact with a polar medium and more particularly water, a compacted conformation.

This results in the creation of a compacted architecture in the form of nanoparticles, in which there are at least partly a GAG molecule entity and at least one hydrocarbon-based radical.

By this way, it is advantageously produced mono-dispersed colloidal suspensions of particles having a mean diameter ranging from 50 to 800 nm, in particular from 75 to 500 nm, and more preferably from 100 nm to 200 nm.

They exhibit a polydispersity index lower than 0.17, more preferably lower than 0.16 and still more preferably lower than 0.15.

The mean particle size and polydispersity index of nanoparticles were determined at 25°C by quasi-elastic scattering (QELS) using a nanosizer (such as Zetasizer Nano 6.12, Malvern Instruments Ltd., UK).

According to an embodiment, the nanoparticles in accordance with the invention present a zeta potential ranging from -75 mV to - 30mV, more preferably from -65mV to - 35mV and still more preferably from -60 mV to -40mV.

In the instant invention, the zeta potential measurement may be carried out by laser Doppler interferometry (for example using a Zetasizer (Zetasizer 4, Malvern Instruments ltd., UK)) according to manufacturer recommendations.

Regarding the zeta potential it is clear that it is linked to the amount of charges of the considered GAG macromolecule which aren't neutralized by cationic groups of the molecules of squalene compound of formula (I).

According to an embodiment, the ratio of positive to negative charge in a bioconjugate suitable for the invention is such that it may have a global charge near the neutrality.

For example, above the threshold of molar ratio Fpx:Sq⁺ of 1:6, the Zeta potential reaches values close to zero. Taking into account that Fpx has ten negative charges, at molar ratios Fpx:Sq⁺ of 1:10 and 1:20, the number of Sq⁺ per Fpx chain is in such case sufficient enough to neutralize the Fpx charges.

According to an embodiment, nanoparticles of the invention may have a positive/negative charges ratio ranging from 10:0.5 to 10:20, more particularly from 10:1 to 10:6.

### a) anionic glycosaminoglycan-type macromolecule and derivatives thereof

According to the instant invention, the term « derivatives » encompasses any organic or inorganic salts, or a solvate of the considered glycosaminoglycan-type macromolecule.

The term anionic (also named negatively-charged) glycosaminoglycan-type macromolecules suitable for the present invention encompasses sulfated and/or carboxylated polyosides such as heparin, fondaparinux, idraparinux, idrabiotaparinux, polysulfated pentosane, sulfated dextran, sulfated heparans, hyaluronan, sulfated dermatan, sulfated chondroitins, danaparoid, and the compound EP217609 (see Olson et al., Blood, 2012, 8; 119(10):2187-95).

In a preferred embodiment, the anionic glycosaminoglycan-type macromolecule is fondaparinux (Fpx).

The term « derivatives » also encompasses a conjugate of a GAG macromolecule with at least one molecule of an active principle, like for example a drug, having a low bioavailability such as paclitaxel. The covalent coupling of the considered active principle with a GAG macromolecule does not raise any difficulty for a man skilled in the art. By this way, the instant invention allows, to exploit the unexpected property of GAG to form nanoparticles with the cationic squalene derivative of formula (I) as defined above, for preparing nanoparticles with molecule(s) of active principle(s) conjugated to at least one GAG macromolecule, i.e. conjugates. The considered conjugated molecule(s) of active principle(s) just need to be covalently linked directly or through a linker to a GAG macromolecule.

Advantageously, the molecule(s) of active principle(s) other than GAG macromolecules may be proteins or peptides. For example, this embodiment could be very useful for insulin or calcitonin oral delivery, knowing the poor bioavailability of these compounds. In another embodiment, antigens or fragments of antigens could be used for vaccination purposes (either systemic or mucosal).

Other hydrophilic compounds or macromolecules with poor oral absorption can be also used for coupling with GAGs.

### b) cationic squalene derivatives

It should be noted that, in the present invention, reference is made, independently, to a "compound" having a squalene or squalenoyl structure.

The term "compound" is intended more specifically to define a cationic compound having a squalene or squalenoyl structure, which, when it reacts with a molecule of active agent, forms an ionic bioconjugate.

For the purpose of the present invention, a compound having a squalene structure may be represented by the formula (I) which follows: 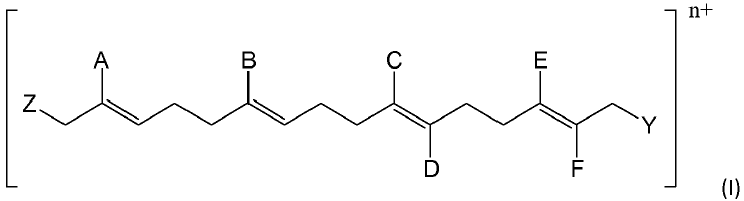 wherein:
- A, B, C, D, E and F are, independently ones from the others, a hydrogen atom or a methyl group,
- Z and Y, independently one from the other, represent a radical 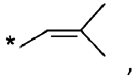 an ethyltrialkylammonium radical, an ethylguanidium radical (also named ethylguanidinium radical) or a 1-[ethylideneamino]guanidinium radical with the proviso that at least one of Z and Y is different from 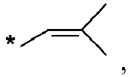 the symbol * showing the position of the attachment of the radical to the structure, and
- n is equal to 1 or 2.

According to a preferred embodiment, n is equal to 1.

According to a preferred embodiment, at least one Y represents one ethyltrialkylammonium radical and n is equal to 1.

According to another embodiment, Y and Z represent one ethyltrialkylammonium radical and n is equal to 2.

According to a preferred embodiment Y and Z are identical and represent respectively one ethyltrialkylammonium radical.

According to the instant invention, an ethyltrialkylammonium radical means a quaternary ethylammonium radical of formula 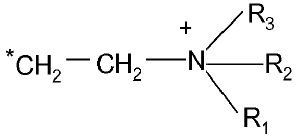 wherein R₁, R₂ and R₃ are independently a C₁₋₁₀ alkyl group, preferably a methyl group, an ethyl group, a propyl group, an isopropyl group, a butyl group, an isobutyl group, a tert-butyl group, a pentyl group, an isopentyl group, an hexyl group, an heptyl group, an octyl group, a nonyl group or a decyl group, more preferably an ethyl group or a methyl group and still more preferably a methyl group, the symbol * showing the position of the attachment of the ethyltrialkylammonium radical to the rest of the structure.

According to the present invention, an ethylguanidinium radical means a group of formula 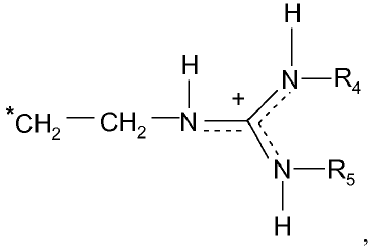 wherein R₄ and R₅ are independently a hydrogen atom or a C₁₋₁₀ alkyl group, preferably a methyl group, an ethyl group, a propyl group, an isopropyl group, a butyl group, an isobutyl group, a tert-butyl group, a pentyl group, an isopentyl group, an hexyl group, an heptyl group, an octyl group, a nonyl group or a decyl group, more preferably an ethyl group or a methyl group and still more preferably a methyl group, the symbol * showing the position of the attachment of the ethylguanidinium radical to the rest of the structure. In a preferred embodiment, R₄ and R₅ are simultaneously a hydrogen atom.

According to the present invention, a 1-[ethylideneamino] guanidinium radical means a group of formula: 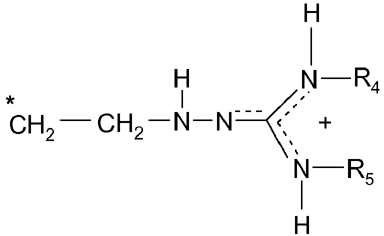 wherein R₄ and R₅ are independently a hydrogen atom or a C₁₋₁₀ alkyl group, preferably a methyl group, an ethyl group, a propyl group, an isopropyl group, a butyl group, an isobutyl group, a tert-butyl group, a pentyl group, an isopentyl group, an hexyl group, an heptyl group, an octyl group, a nonyl group or a decyl group, more preferably an ethyl group or a methyl group and still more preferably a methyl group, the symbol * showing the position of the attachment of the 1-[ethylideneamino]guanidinium radical to the rest of the structure. In a preferred embodiment, R₄ and R₅ are simultaneously a hydrogen atom.

According to another preferred embodiment, cationic compounds of formula (I) are mono or poly-nor squalene derivatives like trisnor or hexanor squalene derivatives.

These compounds are carried out in the case of the present invention under the form of salts and in particular inorganic salts like for example, chloride, sulphate, or alkylsulfonate.

By way of illustration of precursor of cationic squalene compounds capable of forming nanoparticles according to the present invention with a negatively charged glycosaminoglycane macromolecule, mention may more particularly be made of trimethyl(trisnor-squalenyl)ammonium (Sq⁺) and 1,20-trimethyl(hexanorsqualenyl)ammonium (Sq⁺⁺) as disclosed hereafter. 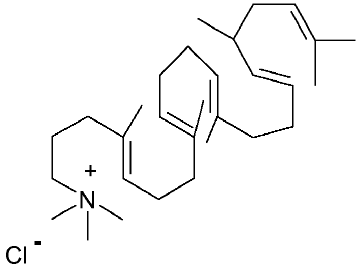 Trimethyl (trisnor-squalenyl)ammonium chloride salt 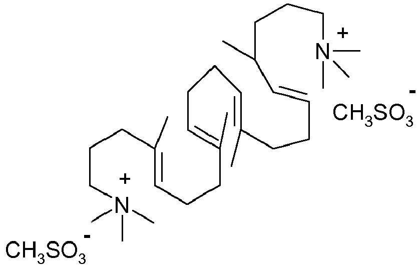 1,20-bis-trimethylammonium(hexanorsqualenyl) dimethanesulfonate salt

A nanoparticle according to the present invention may comprise at least cationic squalene derivative of formula (I) (Sq) as defined above and (GAG) in molar ratio Sq/GAG at least equal to 0.5, in particular ranging from 0.5 to 10.

However, the number of molecule of squalene derivative of formula (I) intended to interact through electrostatic binding with one molecule of GAG may advantageously be greater than 1 and more particularly greater than 2 and for example until 6 like Fpx to Sq⁺ of 1:6.

In a particular embodiment, nanoparticles according to the present invention are nanoparticles of an ionic bioconjugate comprising one molecule of Fpx and from 0.5 to 20 molecules of Sq+ and more preferably from 0.5 to 6 molecules of Sq+ and still more preferably 6 molecules of Sq+. Advantageously, the mean diameter of said nanoparticles is comprised in a range from 100 nm to 200 nm, more preferably from 110 nm to 190 nm, and more preferably from 115 nm to 155 nm, and still more preferably from 145 nm to 155 nm.

In another particular embodiment, nanoparticles according to the present invention are nanoparticles of an ionic bioconjugate comprising one molecule of Fpx and from 0.5 to 6 molecule of Sq++, more preferably from 0.5 to 2 molecule of Sq++ and still more preferably 0.5 molecules of Sq++. Advantageously, the mean diameter of said nanoparticles is comprised in a range from 100 nm to 800 nm, more preferably from 125 nm to 400 nm, and more preferably from 125 nm to 140 nm.

Thus, in a preferred embodiment, nanoparticles according to the present invention are nanoparticles wherein the negatively charged glycosaminoglycan-type macromolecule is fondaparinux and the molecule of a cationic hydrocarbon-based radical of squalene nature is of formula 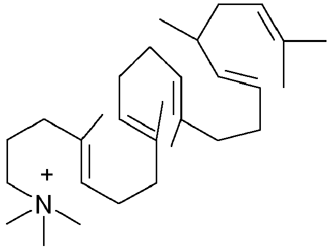 in a molar ratio fondaparinux/cationic hydrocarbon-based radical of squalene of 1:6.

In another preferred embodiment, nanoparticles according to the present invention are nanoparticles wherein the negatively charged glycosaminoglycan-type macromolecule is fondaparinux and the molecule of a cationic hydrocarbon-based radical of squalene nature is of formula 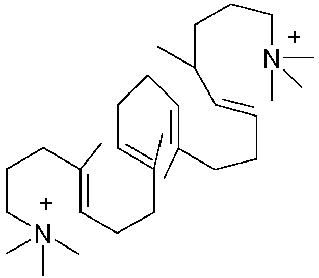 in a molar ratio fondaparinux/cationic hydrocarbon-based radical of squalene of 1:0.5.

As shown in the following experimental part, the nanoparticles of the invention allowed the oral absorption of Fpx with improved pharmacokinetics in a dose-dependent profile. This approach opens up challenging perspectives for the oral delivery of Fpx.

According to one embodiment, the external surface of the nanoparticles of the invention may be further modified with additional moieties chosen among targeting ligands, pharmacokinetic modulating moieties, and mixture thereof.

A pharmacokinetic property is meant a parameter that describes the disposition of an active agent in an organism or a host. Representative pharmacokinetic properties include: drug half-life, hepatic first-pass metabolism, volume of distribution, degree of blood serum protein, e.g. albumin, binding, degree of tissue targeting, cell type targeting, etc.

According to the pharmacokinetic parameter to be modulated, the skilled artisan may select among known molecules in the art, the appropriate molecule that is suitable to obtain the required modulation.

This functionalization of the external surface of the nanoparticles of the invention is particularly interesting when these nanoparticles are dedicated to be administered by systemic route.

According to one embodiment, the targeting ligands may for example be chosen among antibodies, ligands, such as agonists or antagonists of cell surface receptors or molecules able to interact with cell surface proteins or lipids, such as for example mannose or galactose or annexin.

According to one embodiment, the pharmacokinetic modulating moieties that may be used in accordance with the invention may be chosen among amphiphilic derivatives of PEG (polyethylene glycol).

Those PEG derivatives may be preferred to improve stealth properties of the nanocapsules of the invention or transmucosal passage.

As further examples of pharmacokinetic modulating moiety that may be considered in surface of the nanocapsules of the invention, one may also mention non-ionic surface agents, such as polyols or their derivatives, such as for example the poloxalkol Pluronic F68^{®}. According to another embodiment, preferred non-ionic surface active agents are PEG with a dendritic benzyl polyether head, polyoxyethylene alcohols or polyoxyethylene nonylphenylether.

### PREPARATION OF NANOPARTICLES OF THE INVENTION

The formation of nanoparticles described above can be carried out according to conventional techniques insofar as they involve bringing the ionic bioconjugate, formed by electrostatic interaction between the molecule GAG and the squalene compound of formula (I), into contact with an aqueous medium under conditions suitable for its agglomeration in the form of nanoparticles. This may in particular involve methods referred to as nanoprecipitation.

Nanoprecipitation is the most common technique which combines the advantages of a one-step preparation, the easy scale-up and the use of less toxic solvents as compared to other fabrication methods.

More particularly, the nanoparticles according to the present invention may be obtained by a method comprising at least the steps consisting of:
- providing a solution of a cationic squalene compound of formula (I) as defined above in a water-soluble organic solvent,
- pouring said organic solution in an aqueous phase containing at least one molecule of negatively charged GAG macromolecule, the concentration of compound of formula (I) as defined above and of GAG macromolecule being adjusted to get an ionic bioconjugate which, under stirring, instantaneously forms the expected nanoparticles in suspension in said aqueous phase, and, where appropriate, the isolation of said nanoparticles.

Unexpectedly, the inventors observed that cationic squalene compounds alone were not able to self-assemble as nanoparticles in water, but formed aggregates when used alone in the nanoprecipitation technique. However, when used in combination with GAG, the spontaneous formation of nanoparticles in water was observed likely due to electrostatic and hydrophobic interactions between molecules.

Preferably, the cationic squalene compounds are chosen among inorganic salts of trimethyl(trisnor-squalenyl)ammonium (Sq⁺) and 1,20-trimethyl(hexanorsqualenyl)ammonium (Sq⁺⁺).

Particle size is an important parameter determining the *in vivo* fate of the nanoparticles after oral administration and as a rule thumb, sizes smaller than 500 nm are considered to facilitate the interactions with the epithelia.

In this respect, the preparation of nanoparticles is advantageously optimized to obtain stable monodispersed colloidal suspensions together with high drug loadings.

This purpose may be achieved by adjusting, i) the nature of the organic solvent, ii) the volume ratio of the two phases (organic/aqueous phases), and iii) the concentration of the squalene compound.

Thus, the acetone or ethanol appears to be advantageous as potential organic solvent.

The lowest volume ratio organic to aqueous phase of 1:10 was the best to obtain monodispersed nanoparticles with small mean diameter i.e. lower than 200 nm, more particularly when the active principle is Fpx. This ratio has to be adjusted to promote the nanoprecipitation leading to the formation of nanoparticles having the expected low size and advantageously a polydispersity index lower than 0.2.

In a preferred embodiment, nanoparticles according to the present invention are nanoparticles comprising GAG macromolecules and in particular Fpx and Sq+, in a molar ratio of from 1:0.5 to 1:20, more preferably of from 1:0.5 to 1:6 and still more preferably of 1:6.

In another preferred embodiment, nanoparticles according to the present invention are nanoparticles comprising GAG macromolecules and in particular Fpx and Sq++ in a molar ratio of from 1:0.5 to 1:6, more preferably of from 1:0.5 to 1:2 and still more preferably of 1:0.5.

The reaction can generally be carried out at ambient temperature. Irrespective of its value, the reaction temperature should not affect the activity of the active molecule under consideration. The method for preparing the nanoparticles according to the invention is particularly advantageous since it does not require the obligatory presence of surfactants.

This property is particularly beneficial since a large number of surfactants do not prove to be compatible with an *in vivo* application.

However, it is understood that the use of surfactants, generally advantageously free of any toxicity, can be envisioned in the context of the invention. Surfactants of this type may, moreover, make it possible to obtain even smaller sizes during the formation of nanoparticles. By way of non limiting illustration of surfactants of this type which can be used in the present invention, mention may in particular be made of polyoxyethylene-polyoxypropylene copolymers, phospholipid derivatives and lipophilic derivatives of polyethylene glycol.

As a lipophilic derivative of polyethylene glycol, mention may, for example, be made of polyethylene glycol cholesterol and polyethylene glycol squalene. As examples of polyoxyethylene-polyoxypropylene block copolymers, mention may particularly be made of polyoxyethylene-polyoxypropylene-polyoxyethylene triblock copolymers, also known as poloxamers^{®}, pluronics^{®} or synperonics, and which are sold in particular by the company BASF.

Poloxamines, which are related to these families of copolymers, and which consist of hydrophobic segments (based on polyoxypropylene), hydrophilic segments (based on polyoxyethylene) and a central part deriving from the ethylenediamine unit, can also be used.

### GALENIC FORMULATIONS OF NANOPARTICLES ACCORDING TO THE INVENTION

As indicated above, the present invention is also directed to the use of at least one nanoparticle according to the invention in pharmaceutical or dermatological compositions.

Thus, another aspect of the instant invention relates to a pharmaceutical or dermatological composition comprising, as active material, at least nanoparticles according to the present invention, optionally in the form of a lyophilisate, and/or capsules, in combination with at least one acceptable pharmaceutical vehicle.

In a particular embodiment, a pharmaceutical or dermatological composition according to the invention comprises further a P-glycoprotein inhibitor which is chosen among verapamil, quinidine, amiodarone, reversin, matairesinol, sipholenol and cyclosporin.

The nanoparticles in accordance with the present invention may be combined therein with at least one pharmaceutically acceptable vehicle.

The nanoparticles in accordance with the present invention can be administered by any of the conventional routes.

By way of examples of pharmaceutical formulations compatible with the nanoparticles according to the invention, mention may in particular be made of:
- intravenous injections or infusions;
- saline solutions or solutions of purified water;
- compositions for inhalation;
- creams, ointments, lotions, gels;
- capsules, sugar-coated tablets, cachets and syrups incorporating in particular, as vehicle, water, calcium phosphate, sugars, such as lactose, dextrose or mannitol, talc, stearic acid, starch, sodium bicarbonate and/or gelatin.
Preferably, said pharmaceutical composition may be orally administered, systemically administered or topically administered and more preferably orally administered.

In effect, given their small size, the nanoparticles of the invention can be administered intravenously in the form of an aqueous suspension and are therefore compatible with the vascular microcirculation.

When the nanoparticles are used as a dispersion in an aqueous solution, they may be combined with excipients such as sequestering or chelating agents, antioxidants, pH regulators and/or buffering agents.

According to another specific embodiment, the nanoparticles according to the invention are in the form of a lyophilisate.

Advantageously, the previously disclosed method for preparing said nanoparticles may thus comprise a subsequent lyophilization step, particularly suitable for being able to formulate a solid form.

According to a specific embodiment, the solid form dosage is a delayed release solid form dosage like for example enteric coated tablets or capsules and which surface coating provides the delayed release.

Thus, the present invention also relates to a lyophilisate comprising at least nanoparticles as described above.

Another aspect of the present invention is a solid form dosage containing at least nanoparticles as defined above, optionally in the form of a lyophilisate.

The drying of nanoparticles is advantageously performed with a cryoprotectant to protect the nanoparticles fragile structure against the mechanical stress due to ice crystals formation. Mono-, di- or poly-saccharides like glucose, glycerol and trehalose are commonly used cryoprotectants forming a glassy matrix upon water sublimation. As shown in the following experimental part, trehalose is particularly advantageous.

For an administration by oral route, the nanoparticles of the invention, under a freeze-drying form or not, may be also formulated in a controlled-release solid dosage form.

Such solid dosage forms may be particularly advantageous for obtaining a wide range of release profiles and a reduction in bio-variability.

In particular, a pH resistant solid dosage forms are particularly useful for improving the absolute bio-availabilities of the nanoparticles of the invention with respect to the acidic pH of the stomach.

Gelatin capsule coated with an enteric coating may be for example a convenient controlled-release solid dosage form.

This coating is preferably achieved by immersing the capsule, incorporating nanocapsules of the invention, in an aqueous solution of the considered enteric polymer.

Such a polymer may be selected among polyvinyl acetate phthalate (PVAP), hydroxypropylmethyl-cellulose acetate succinate (HPMCAS), cellulose acetate phthalate (CAP), methacrylic acid copolymer, hydroxy propyl methylcellulose succinate, cellulose acetate succinate, cellulose acetate hexahydrophthalate, hydroxypropyl methylcellulose hexahydrophthalate, hydroxypropyl methylcellulose phthalate (HPMCP), cellulose propionate phthalate, cellulose acetate maleate, cellulose acetate trimellitate, cellulose acetate butyrate, cellulose acetate propionate, methacrylic acid/methacrylate polymer (acid number 300 to 330 and also known as EUDRAGIT L), which is an anionic copolymer based on methacrylate and available as a powder (also known as methacrylic acid copolymer, type A NF, methacrylic acid-methyl methacrylate copolymer, ethyl methacrylate-methylmethacrylate-chlorotrimethylammonium ethyl methacrylate copolymer, and the like, and combinations comprising one or more of the foregoing enteric polymers. Other examples include natural resins, such as shellac, SANDARAC, copal collophorium, and combinations comprising one or more of the foregoing polymers. Yet other examples of enteric polymers include synthetic resin bearing carboxyl groups. The methacrylic acid: acrylic acid ethyl ester 1:1 copolymer solid substance of the acrylic dispersion sold under the trade designation "EUDRAGIT L-100-55" may be suitable.

In addition to the abovementioned compounds, the pharmaceutical or dermatological compositions according to the invention may contain agents such as preservatives, wetting agents, solubilizing agents and colorants.

The pharmaceutical compositions of the invention may further contain other active agents of which it may be beneficial to take advantage from a therapeutic point of view, together with the effect of the GAG.

By way of representation of these active materials that may be combined with the nanoparticles in accordance with the present invention, mention may in particular be made of active agents, such as amlodipine, gallopamil, verapamil, barnidipine, felodipine, isradipine, lacidipine, lercanidipine, nicardipine, nifedipine, nimodipine, nisoldipine, nitrendipine or diltiazem. Preferably, the active agent is an inhibitor of Pgp (P-glycoprotein) and is chosen among verapamil, quinidine, amiodarone, reversin, matairesinol, sipholenol and cyclosporin, and still more preferably the active agent is verapamil.

For obvious reasons, the amounts of bioconjugate and/or nanoparticle according to the invention that can be used are capable of varying significantly according to the method of use and the route selected for their administration.

The examples which follow illustrate the present invention without, however, being limited thereto.

### Figures

Figure 1: is a diagram which presents the encapsulation efficiencies (EE) of Fpx within the Fpx:Sq⁺ NPs with different ratios.
Figure 2A: is a diagram which shows the plasma-concentration-time profile of free Fpx as compared to encapsulated Fpx after intravenous administration.
Figure 2B: is a diagram which shows the plasma-concentration-time profile of free Fpx as compared to encapsulated Fpx after oral administration.
Figure 3: is a diagram showing a comparison of the permeation of Fpx across the intestinal tissue under specific conditions as explained in the experimental part.
Figure 4: is a diagram which shows, in case of Fpx:Sq+ NPs, the amounts of Fpx which permeates through the intestinal epithelium in presence or not of verapamil added in the serosal side.
Figure 5: is a diagram showing net fluxes of Fpx in free form or in Fpx:Sq+ NPs in presence or not of verapamil when added in serosal side at 180 min.
Figure 6: is a diagram which shows the plasma-concentration-time profile of free Fpx as compared to encapsulated Fpx filled in enteric-coated capsules after oral administration.

### EXAMPLES

### 1. Materials and methods

### 1.1. Materials

The infrared spectra are obtained by measurement on a pure solid or liquid using a Fourier spectrometer (Bruker Vector^{®} 22 Fourier Transform spectrometer). Only the significant absorptions are noted.

The optical rotations were measured using a Perkin-Elmer^{®} 241 polarimeter, at a wavelength of 589 nm.

The ¹H and ¹³C NMR spectra were recorded using a Bruker ARX^{®} 400 spectrometer (at 400 MHz and 100 MHz, respectively, for ¹H and ¹³C) or a Bruker Avance^{®} 300 spectrometer (at 300 MHz and 75 MHz, respectively, for ¹H and ¹³C).

The mass spectra were recorded using a Bruker Esquire-LC^{®} instrument. The thin layer chromatography analysis was carried out on silica 60F₂₅₄ plates (layer of 0.25 mm).

The column chromatography was carried out on silica 60 gel (Merck, 230-400 mesh ASTM).

The mean particle size and polydispersity index of nanoparticles were determined at 25°C by quasi-elastic light scattering (QELS) using a nanosizer (Zetasizer Nano 6.12, Malvern Instruments Ltd., UK). The measures were performed in triplicate after dilution (1/50) of the NPs with MilliQ^{®} water. Nanoparticles of 150 nm were obtained with a PDI less than 0.1.

The zeta potential was determined using a Zetasizer (Zetasizer 4, Malvern Instruments ltd., UK) after 1/50 dilution of 1 mL of the NPs suspensions in 1 mL of an aqueous solution of KCl (1 mM).

All the reactions using compounds sensitive to air or to water were carried out under a nitrogen atmosphere.

Fondaparinux (Fpx, Arixtra^{®}, 10 mg/0.8 mL) was purchased from GlaxoSmithKline (UK).

Sodium phosphate dibasic, sodium phosphate monobasic, verapamil and azure A were obtained from Sigma-Aldrich Chemical Co. (France).

Acetone was purchased from Carlo Erba (Italy) and Alexa Fluor 568 from Life Technologies (France). Filtered MilliQ water (Millipore^{®}, France) was used.

Male Sprague-Dawley rats (250 - 300 g) were obtained from Janvier SAS (France).

### EXAMPLE 1: Synthesis of trimethyl(trisnor-squalenyl)ammonium chloride salt (Sq⁺)

### 1.1 Synthesis of (4E,8E,12E,16E)-4,8,13,17,21-pentamethyldocosa-4,8,12,16,20-pentaen-1-yl methanesulfonate

To a stirred solution of trisnorsqualene alcohol (582 mg, 1.5 mmol) (trisnorsqualene alcohol was prepared by sodium borohydride reduction of the corresponding aldehyde obtained by periodic acid cleavage of 2,3-epoxysqualene according to van Tamelen (Tetrah. Lett., 1962, 121-124)) in anhydrous CH₂Cl₂ (7 mL) at 0°C, was added DMAP (10 mg) and dropwise, triethylamine (224 mg, 2.2 mmol) followed by methanesulfonyl chloride (205 mg, 1.8 mmol). The mixture was then slowly raised to room temperature and stirred for 2 h. The reaction was then quenched with brine and the mixture was extracted with CH₂Cl₂ (4 × 50 mL). The combined organic extracts were washed with brine, dried over MgSO₄ and concentrated in vacuo. The crude 1,1',2-trisnorsqualenyl methanesulfonate (695 mg, 85 %) was used directly without further purification.
¹H NMR (300 MHz, CDCl₃) δ: 5.14-4.96 (m,5H, CH *vinyl*), 4.12 (t, 2H, *J* = 6.5 Hz, C*H*₂O), 2.91 (s, 3H, C*H*₃SO₂), 2.08-1.90 (m,18H, CH₂), 1.90-1.76 (m, 2H, CH₂, MsOCH₂C*H*₂), 1.58 (s, 3H, HC=C(C*H*₃)₂), 1.53 (s, 3H, 15H, HC=C(C*H*₃)CH₂)
¹³C NMR (300 MHz, CDCl₃) δ: 135.1 (Cq), 134.9 (Cq), 134.8 (Cq), 132.8 (Cq), 131.2 (Cq), 125.8 (CH), 124.5 (CH), 124.4 (CH), 124.2 (2 CH), 69.7 (CH₂, *C*H₂OMs), 39.7 (2 CH₂), 39.6 (CH₂), 37.3 (CH₃, OSO₂*C*H₃), 35.1 (CH₂), 28.2 (2 CH₂), 27.2 (CH₂), 26.7 (CH₂), 26.6 (CH₂), 26.5 (CH₂), 25.6 (CH₃, HC=C(CH₃)₂), 17.6 (CH₃), 16.1 (CH₃), 16.0 (3 CH₃), 15.8 (CH₃)

### 1.2 Synthesis of (6E,10E,14E,18E)-22-Chloro-2,6,10,15,19-pentamethyldocosa-2,6,10,14,18-pentaene

To a stirred solution of trisnorsqualenyl methanesulfonate (500 mg, 1.08 mmol) in anhydrous DMF (5 mL) was added LiCl (456 mg, 10.8 mmol). The reaction mixture was heated at 80°C for 2 h. After cooling to room temperature, the mixture was concentrated under reduced pressure. The residue was taken in water then extracted with diethyl ether (5 × 12 mL). The combined organic phases were dried over MgSO₄, filtered and concentrated *in vacuo.* The crude product was purified by chromatography on silica gel (cyclohexane/EtOAc, 98:2 v/v) to give the 1-chlorotrisnorsqualene as a colorless oil (377 mg, 86 %).
IR (neat, cm⁻¹) ν = 2855-2970, 1441, 980, 832
¹H NMR (300 MHz, CDCl₃) δ: 5.25-5.02 (m, 5H, CH *vinyl),* 3.56 (t, 2H, J = 6.7 Hz, C*H*₂Cl), 2.20-1.91 (m, 18H, CH₂), 1.85-1.90 (m, 2H, ClCH₂C*H*₂), 1.61 (s, 3H, HC=C(C*H*₃)₂), 1.53 (m, 15H, HC=C(C*H*₃)CH₂)
¹³C NMR (300 MHz, CDCl₃) δ: 135.1 (Cq), 134.9 (Cq), 134.8 (Cq), 133.0 (Cq), 131.2 (Cq), 125.6 (CH), 124.5 (CH), 124.4 (CH), 124.3 (2 CH), 44.5 (CH₂, CH₂Cl), 39.8 (2 CH₂), 39.7 (CH₂), 39.6 (CH₂), 36.6 (CH₂), 30.8 (CH₂), 28.3 (2 CH₂), 26.8 (CH₂), 26.7 (CH₂), 26.6 (CH₂), 25.7 (CH₃, HC=C(CH₃)₂), 17.7 (CH₃), 16.0 (CH₃), 15.9 (2 CH₃), 15.8 (CH₃)

### 1.3 Synthesis of Trimethyl[(4E,8E,12E,16E)-4,8,13,17,21-pentamethyldocosa-4,8,12,16,20-pentaen-1-yl]azanium chloride

A 50 % aqueous sodium hydroxyde solution was (20 mL) was added dropwise by mean of a dropping funnel to trimethylamine hydrochloride (3.0 g, 31.4 mmol) placed in a distilled flask. The evolved trimethylamine gas was passed through a washed bottle containing sodium hydroxide pellets and allowed to bubble through 4.0 g of anhydrous ethanol placed in another wash bottle and exactly weighted. The obtained Me₃N (2.2 g, 37.2 mmol) ethanol solution was added to 1-chlorotrisnorsqualene (300 mg, 0.71 mmol) in a screw cap sealed tube equipped with a stirred bar. The reaction mixture was stirred at 100°C for 72 h. After cooling to room temperature, the mixture was concentrated under reduced pressure to provide the tertiary ammonium salt as a pale yellow oil (294 mg, 98 %). The crude product was used without further purification.
¹H NMR (300 MHz, [D4] MeOH) δ: 5.26 (t, *J* = 6.0 Hz, 1H, CH *vinyl),* 5.25-5.15 (m, 4H, CH *vinyl),* 3.40-3.30 (m, 2H, Me₃NC*H*₂), 3.21 (s, 9H, (C*H*₃)₃N⁺), 2.20-1.80 (m, 20H, CH₂), 1.69 (s, 6H, CH₃),1.62 (s, 12H, CH₃)
¹³C NMR (300 MHz, [D4] MeOH) δ: 136.8 (2Cq), 1.36.7 (Cq), 134.7 (Cq), 132.8 (Cq), 128.1 (CH), 126.45 (CH), 126.4 (CH), 126.3 (2CH), 68.5 (CH₂, CH₂NMe₃), 54.5 (CH₃, N(*C*H₃)₃), 54.45 (CH₃, N(*C*H₃)₃), 54.4 (CH₃, N(*C*H₃)₃), 41.7 (2CH₂), 41.6 (CH₂), 37.9 (CH₂), 30.1 (2CH₂), 28.7 (CH₂), 28.4 (CH₂), 26.8 (CH₃, HC=C(*C*H₃)₂), 23.1 (CH₂), 18.7 (CH₃), 17.1 (3CH₃), 16.7 (CH₃)
MS (ESI): *m*/*z* (%) = 428 (100)
Anal. Calcd for C₃₀H₅₄ClN (%): C 77.62, H 11.73, N 3.02. Found: C 77.21, H 11.71, N 2.90

### EXAMPLE 2: Synthesis of 1,20-bis-trimethylammonium (hexanorsqualenyl) dimethanesulfonate salt (Sq⁺⁺)

### 2.1 Synthesis of (4E,8E,12E,16E)-20-(Methanesulfonyloxy)-4,9,13,17-tetramethylicosa-4,8,12,16-tetraen-1-yl methanesulfonate

To an ice-cooled stirred solution of 1.20-bis-trisnorsqualene alcohol (1.2 g, 3.30 mmol) (1.20-bis-trisnorsqualene alcohol -also named 1.20-hexanorsqualene diol- is available from squalene (SQ) via 2,3,22,23-dioxidosqualene (Sen et al., J. Am. Chem. Soc., 1989, 11, 1508-1510)) and few crystals of DMAP (10 mg) in anhydrous CH₂Cl₂ (20 mL) was added dropwise triethylamine (1.27 mL, 9.90 mmol) followed by methanesulfonyl chloride (564 µl, 7.92 mmol). The reaction mixture was stirred at room temperature for 4h and water (40 mL) was added. The mixture was extracted with CH₂Cl₂ (4 × 30 mL). The combined organic extracts were washed with brine, dried over MgSO₄ and concentrated in vacuo. The residue was purified by flash chromatography on silica gel (AcOEt/cyclohexane 1:2) to provide pure bis-mesylate as colorless oil (800 mg, 67 %).
¹H NMR (300 MHz, CDCl₃) δ: 5.09-5.06 (m, 4H, CH *vinyl),* 4.10 (t, 4H, J = 6.5 Hz, C*H*₂OMs), 2.93 (s, 6H, C*H*₃SO₂), 2.05-1.89 (m, 16H, CH₂), 1.73-1.82 (m, 4H, C*H*₂CH₂OMs), 1.53 (s, 12H, HC=C(*C*H₃)CH₂)

### 2.2 Synthesis of trimethyl[(4E,8E,12E,16E)-4,9,13,17-tetramethyl-20-(trimethylazaniumyl)icosa-4,8,12,16-tetraen-1-yl]azanium dimethanesulfonate

A solution of Me₃N (4.0 g, 67.7 mmol) in ethanol (9 mL) was added to bis-trisnorsqualenylmethanesulfonate (650 mg, 1.22 mmol) in a screw cap sealed tube equipped with a stirred bar. The reaction mixture was stirred at 100 °C for 5 days and treated as described above for compound trimethyl[(4*E*,8*E*,12*E*,16*E*)-4,8,13,17,21-pentamethyldocosa-4,8,12,16,20-pentaen-1-yl]azanium chloride (Sq+) to yield salt Sq++ (600 mg, 92 %).
¹H NMR (300 MHz, [D4]MeOH ) δ: 5.09-5.29 (m, 4H, CH vinyl), 3.29-3.42 (m, 4H, CH2), 3.18 (s, 18H, J = 6.7 Hz, CH₃N+), 2,84 (s, 6H, CH₃S), 2.01-2.33 (m, 18H, CH2), 1.82-1.89 (m, 4H, CH₂), 1.41-1.76 (m, 12H, CH₃)
Anal. Calcd for C₃₃H₆₈N₂O₆S₂ (%): C 60.15, H 10.41, N 4.38, O 15.02, S 10.04. Found: C 60.06, H 10.50, N 4.38, O 15.62, S 9.03

### EXEMPLE 3: Coupling of Fpx with a fluorophore, Alexa Fluor 568

Fpx was coupled with fluorochrome (Alexa Fluor 568) at the molar ratio 1:1 in order to evaluate the passage of Fpx loaded in squalenoyl NPs through intestinal epithelium. In presence of the fluorochrome, the physicochemical parameters of NPs were not significantly modified at the molar ratio 1:1.

Briefly, 10 mg of Fpx was dissolved in 1 mL of a bicarbonate buffer pH 8.5. 4.6 mg of Alexa Fluor 568 was added to the reaction mixture and stirred at room temperature for 6 h. Then, the Fpx-Alexa Fluor 568 aqueous solution was dialysed against MilliQ water for 6 days and then lyophilized to obtain a pink powder. The crude product was characterized by ¹H NMR (data not shown).

### EXAMPLE 4: Preparation of Fpx: Sq⁺ nanoparticles (NPs) and of Fpx: Sq⁺⁺ nanoparticles (NPs)

Nanoparticles based on the formation of an ion-pair bioconjugate between CSq and polyanionic Fpx were prepared by nanoprecipitation.

Fpx:Sq⁺ NPs at the considered ratio (Fpx to Sq⁺) were prepared by nanoprecipitation method. The considered amount of Sq⁺ (from 0.33 to 13.43 mg/mL) was dissolved in 0.1 mL of acetone and added drop-wise under gentle magnetic stirring into an aqueous solution of Fpx (2.5 mg/mL). Nanoparticles formation spontaneously occurred. Acetone was then evaporated using a Rotavapor^{®} (Buchi, France) and the nanoparticulate suspensions were stored at 4°C until further studies.

In a similar manner, nanoparticles of Fpx:Sq++ (Fpx:Sq++ NPs) were prepared.

The morphology of the so-obtained Fpx:Sq⁺ NPs was investigated by cryo-TEM. The Fpx:Sq⁺ NPs had a regular spherical shape with an inner "onion-type" structure.

The following table 1 shows physiochemical characterization of the Nps of Fpx:Sq+ and Fpx:Sq++ with different molar ratios.

**Table 1**

| **Type of NPs** | **Molar ratio** | **Charge ratio** | **z[mV]** | **d [nm]** | **PDI** |
|---|---|---|---|---|---|
| Fpx:Sq⁺ | 1:0.5 | 10:0.5 | -54 ± 5 | 118 ± 2 | 0.12 ± 0.01 |
| | 1:1 | 10:1 | -61 ± 5 | 129 ± 1 | 0.15 ± 0.01 |
| | 1:2 | 10:2 | -61 ± 1 | 139 ± 1 | 0.14 ± 0.03 |
| | 1:3 | 10:3 | -67 ± 4 | 145 ± 3 | 0.14 ± 0.01 |
| | 1:4 | 10:4 | -58 ± 4 | 146 ± 5 | 0.15 ± 0.02 |
| | 1:5 | 10:5 | -60 ± 1 | 143 ± 3 | 0.12 ± 0.02 |
| | 1:6 | 10:6 | -60 ± 3 | 149 ± 1 | 0.13 ± 0.01 |
| Fpx:Sq⁺⁺ | 1:0.5 | 10:1 | -52 ± 10 | 133 ± 3 | 0.14 ± 0.02 |

### EXAMPLE 5: Encapsulation efficiency of the Fpx:Sq⁺ nanoparticles which were prepared according to example 4

The azure A assay is the method that has been considered to determine the amount of non-associated Fpx in the supernatants. This azure A assay is a well known method which is more particularly disclosed for example in Hirsjärvi et al. (Eur. J. Pharm. Biopharm., 2010, 76, 200-207).

Azure A was obtained from Sigma-Aldrich Chemical Co. (France). Azure A was dissolved in water (0.04 mg/mL) and 100 µL of this solution was added to 40 µL of sample into 96-well plate. The optical density of the solution was measured with a microplate spectra PR 2100 reader at 490 nm and the extinction was related to the Fpx concentration using a calibration curve.

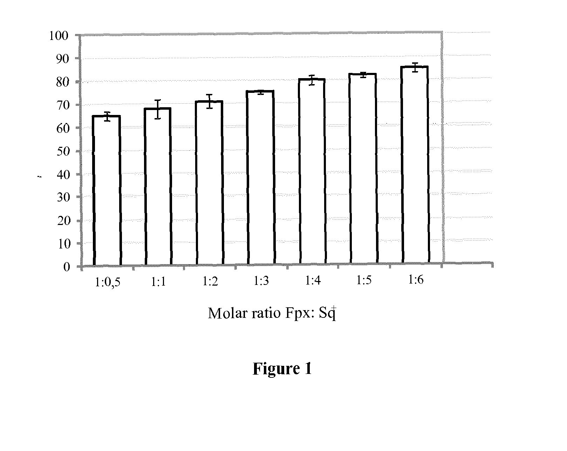

Figure 1 presents the encapsulation efficiencies (EE) obtained with this assay. Fpx concentration is of 2.5 mg/mL and data represent mean values ± standard deviation (n = 6). Best EE were obtained with molar ratios (Fpx to Sq⁺) of 1:6 and 1:10, showing that 85 % and 87 %, respectively, of Fpx with Sq⁺ under the form of NPs as prepared in example 4. Calculated corresponding loadings reached 38.5 % at the ratio 1:6, which is the optimized ratio.

Regarding Fpx with Sq⁺⁺ under the form of NPs as prepared in example 4, calculated corresponding loadings reached 22 % at the ratio 1:0.5, which is the optimized ratio.

The results were confirmed by a direct method (elemental analysis).

### EXAMPLE 6 : Preparation of Fpx-Alexa:Sq⁺ NPs

The preparation of the NPs was done using the method described above. Sq+ (8 mg) was dissolved in 0.1 mL acetone and added drop-wise under gentle magnetic stirring into 1 mL of aqueous solution of Fpx and Fpx-Alexa 568 in molar ratio 1:1 (5 mg/mL). Nanoparticles formation spontaneously occurred. Acetone was then evaporated using a Rotavapor® (Buchi, France) and the nanoparticulate suspensions were stored at 4°C until further studies

### EXAMPLE 7: In vivo anticoagulant activity

The anticoagulant activity of Fpx:Sq⁺ aqueous nanoparticulate suspensions of example 4 was evaluated *in vivo* after oral and intravenous (IV) administration in male Sprague-Dawley rats. The following formulations were administrated orally to the rats (n=4) with the help of feeding needle (Harvard Apparatus, France): NPs at the optimized ratio 1:6 at 25 mg/kg (eq. Fpx) and 50 mg/kg (eq. Fpx), Fpx saline solution at 50 mg/kg and isotonic sodium chloride solution as control. The volume of the administrated solution was 1 mL per 100 g of the rat weight. Blood samples were periodically taken from the jugular vein after anesthesia with isoflurane and the plasma concentration of active Fpx was estimated using an anti-FXa assay. Additionally, a saline solution of Fpx and NPs at the optimized ratio 1:6, both at 200 µg/kg (eq. Fpx) were injected intravenously to the rats (n=3) through the tail vein after anesthesia with isoflurane. Blood samples were periodically taken and the plasma concentration of active Fpx was estimated using an anti-FXa assay.

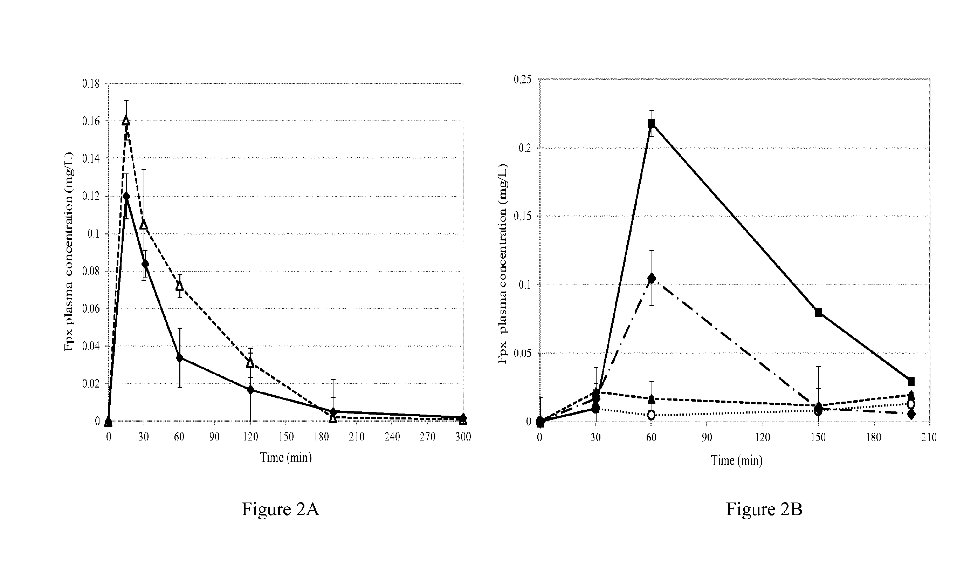

Are shown in figures 2A et 2B, plasma Fpx concentration versus time profiles of rats (A) after intravenous administration of saline solution of Fpx (200 µg/kg) **(--Δ--)** or aqueous suspension of NPs (200 µg/kg) ( 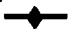 ) (n=3 for each studied group) and after oral administration of saline solution of Fpx (50 mg/kg) **(** 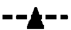 **),** aqueous suspension of NPs at 25 mg/kg ( 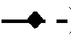 ) and 50 mg/kg ( 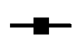 ) and control solution (····o···) (n=4 for each studied group).

Figure 2A shows the plasma concentration-time profile of free Fpx (saline solution) as compared to encapsulated Fpx after IV administration. Both profiles were very similar, with maximum plasma concentration (Cₘₐₓ) of 0.16 mg/L and 0.12 mg/L for free Fpx and encapsulated Fpx, respectively. These results show that the Fpx associated to the NPs may be released quickly in the blood, resulting in an anticoagulant activity similar to free Fpx. These data prove that Fpx could be effectively dissociated from the Sq⁺ moieties in the blood stream.

Figure 2B shows the plasma concentration-time profile of free Fpx (saline solution) as compared to encapsulated Fpx after oral administration. As expected, Fpx in saline solution is not absorbed, the concentration-time profiles being similar to the control group. On the contrary, administration of nanoparticulate suspensions at two distinct concentrations resulted in an increase in plasma concentration of Fpx (Figure 2B). The NPs containing Fpx doses of 25 mg/kg and 50 mg/kg show Cₘₐₓ of about 0.11 mg/L and 0.22 mg/L, respectively with a time to maximum plasma concentration (Tₘₐₓ) of around 60 min in both cases. These results reveal a dose-dependent increase in Cₘₐₓ whereas the time to reach maximal concentration (Tₘₐₓ) is dose-independent. Noteworthy, the Cₘₐₓ obtained after oral administration of 50 mg/kg Fpx:Sq⁺ is close to the prophylactic plasma concentration needed in the venous thromboembolism.

Accordingly, the Fpx:Sq+ nanoparticulate system effectively improves the oral delivery of Fpx as compared to the free Fpx.

### EXAMPLE 8: Comparison of passage of Fpx in solution and of Fpx:Sq⁺ NPs prepared in example 4 through the intestinal epithelium at 37°C in presence or not of verapamil.

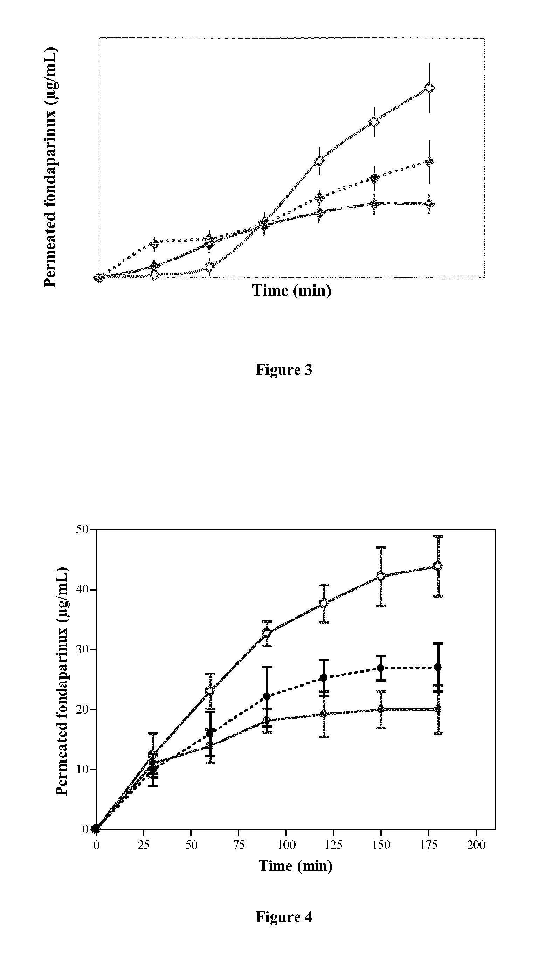

The permeation of Fpx in solution was investigated through the intestinal epithelium at 37°C, in the two directions (mucosal-to-serosal, M-S, absorptive direction and serosal-to-mucosal, S-M, secretory direction) and in presence or not of verapamil, an inhibitor of P-gp (P-glycoprotein) (Fig. 3).

Ussing-type chambers were used to evaluate the permeation of Fpx in fresh intestinal tissues, under the free form and loaded in NPs. For permeation studies non fasting male Sprague Dawley rats weighting 250 to 300 g were used. After sacrificing the rats, jejunum from fresh small intestine was excised and rinsed with chilled physiological solution (isotonic Sorensen's Phosphate buffer solution: 0.2 mM Na₂HPO₄; 0.2 mM NaH₂PO₄, H₂O with a pH of 6.8). The excised intestine was cut into segments of 2 - 3 cm length and mounted in Ussing chamber (0.75 cm² surface area) bathed with Sorensen buffer pH 6.8 in mucosal and serosal sides. The system was maintained at 37°C and continuously oxygenated with O₂/CO₂ (95% /5%). After equilibration at the temperature of the experiment (15 min), Fpx in solution or loaded in NPs at the concentration of 2 mg/mL were added to the donor compartment. For this purpose, 1.2 mL of Fpx:Sq⁺ NPs (at Fpx concentration of 5 mg/mL were diluted in 1.8 mL of Sorensen buffer pH 6.8 prior to the addition to the donor chamber. After 0, 30, 60, 90, 120, 150 and 180 min, 500 µL samples were taken out from the acceptor chambers and replaced with the same volume of fresh transport medium pre-equilibrated at 37°C. The samples were stored at 4°C until analysis. Fpx was quantified by the Azure A assay and Anti-Xa assay. The influence of verapamil was investigated on the intestinal permeation of Fpx in solution and loaded in NPs. For this purpose, verapamil was added to the Sorensen buffer at the concentration of 0.1 mM in the serosal compartment five minutes before the experiment.

Thus, figure 3 is a diagram showing a comparison of the permeation of Fpx across the intestinal tissue under the following experimental conditions: mucosal-to-serosal direction (M-S, continuous lines); serosal-to-mucosal direction (S-M, broken line) and in presence (open symbols) or in absence (filled symbols) of verapamil. Donor chambers contained initially 2 mg/mL of Fpx. Data represent means ± SEM of at least six independent experiments.

It appears that Fpx is poorly transported through the rat intestine, with a maximal permeated concentration of 4.5 µg/mL at 120 min in the M-S direction. When the Fpx passage was studied from the S-M direction, the concentration of the permeated drug is slightly higher than that observed in the M-S direction (7.8 µg/mL as compared to 4.9 µg/mL after 180 min of permeation). However, no statistical difference is observed between the two directions. On the contrary, when verapamil is added in the receptor compartment, its presence increased significantly the transport of Fpx. For example, permeated Fpx concentrations reached 12.6 µg/mL after 180 min of passage in the M-S direction (Figure 3).

Further, figure 4 is a diagram which shows, in case of Fpx:Sq+ NPs, the amounts of Fpx which permeates through the intestinal epithelium in presence or not of verapamil added in the serosal side and more particularly, the time course of passage of Fpx loaded in Fpx:Sq⁺ NPs in absence (continuous line and filled symbols) or in presence of verapamil (continuous line and open symbols) and after renewal of the NPs suspension after 75 min in absence of verapamil (dotted line and filled symbols). Donor chambers contained 2 mg/mL of Fpx and Sq⁺ in molar ratio Fpx to Sq⁺ 1:6. Data represent means ± SEM of at least six independent experiments.

Fig. 4 also shows for Fpx:Sq⁺ NPs, the amounts of Fpx which permeated through the intestinal epithelium in presence or not of verapamil added in the serosal side.

Interestingly, in absence of verapamil, the passage of Fpx increased with time within the first 120 min, with a Fpx concentration reaching around 19 µg/mL. This represents a 4-fold increase as compared to free Fpx (Fig. 4) showing the interest of using nanoparticulate carriers to improve the translocation of Fpx through the intestinal barrier.

Furthermore, in the presence of verapamil, the passage of Fpx after incubation of Fpx:Sq⁺ NPs was increased with an enhancement ratio close to 3, as compared to the same experiment but performed in the absence of verapamil (Fig. 4).

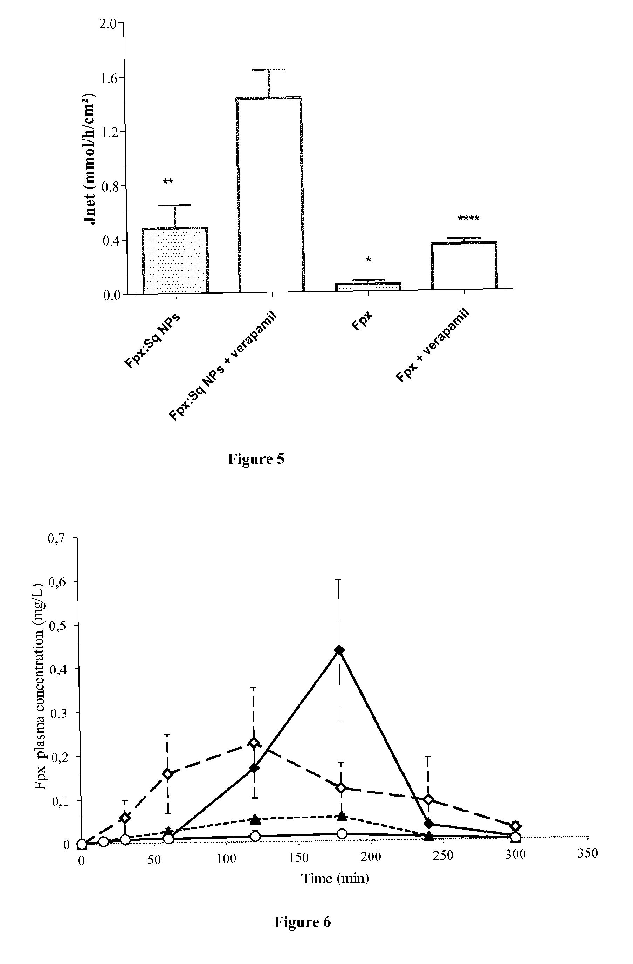

At last, figure 5 represents a diagram showing net fluxes of Fpx in free form or in Fpx:Sq+ NPs in the presence or in the absence of verapamil when added in serosal side at 180 min. Values are indicated as means SEM at least six experiments. (** p< 0.05) Fpx:Sq⁺ NPs vs Fpx:Sq NPs + verapamil; (* p< 0.05) Fpx vs Fpx:Sq⁺ NPs; (**** p<0.05) Fpx + verapamil vs Fpx.

The free Fpx fluxes are increased by a factor of 4.2 in the presence of verapamil. Furthermore, Fpx fluxes are increased by a factor of 9.6 using Fpx-Sq⁺ NPs. The highest fluxes (1.45 mmol/h/cm²) were obtained with Fpx-Sq⁺ NPs in the presence of verapamil.

### EXAMPLE 9: Preparation and characterization of freeze-dried Fpx:Sq+ nanoparticles

The freeze-dried NPs of Fpx:Sq+, previously prepared according to example 4, were prepared by nanoprecipitation. To prevent aggregation, a cryoprotectant which is trehalose, was used at a concentration of 20%w/w during the freeze-drying process. In order to avoid the NPs dilution, trehalose was directly dissolved in the aqueous Fpx solution to reach a concentration of 5 mg/mL. At this aqueous solution, Sq⁺ (8 mg) dissolved in 0.1 mL acetone was added drop-wise under stirring (500 rpm). NPs formation immediately occurred. Acetone was then evaporated using a Rotavapor^{®}. 1 mL of nanoparticulate suspension was then incorporated in glass vials with flat bottom and frozen at -18°C for 15 h. Vials containing the frozen nanoparticulate dispersions were lyophilized using an Alpha 1-2 LO plus freeze-drier to obtain the freeze-dried Fpx:Sq⁺ NPs in powder form. The mean particle size and the PDI of the freeze-dried NPs after rehydration were measured by QELS using a nanosizer (Zetasizer Nano 6.12, Malvern Instruments Ltd., UK). The mean particle size is 198±1 nm and the PDI is 0.12±0.03.

### EXAMPLE 10: Preparation of Fpx:Sq⁺ NPs-filled enteric-coated capsules

The formulation of lyophilized Fpx:Sq⁺ NPs prepared according to example 9 was incorporated within hard gelatin capsules size 9el suitable for use in rats using a manual capsule-filling device (Harvard Apparatus, France). The NPs-filled capsules were then coated with the enteric coating polymer, Eudragit L100^{®} to achieve the gastroresistance characteristics. Briefly, the capsules were immersed in a 15 % (w/v) isopropanol solution of Eudragit^{®} L100. Two procedures were investigated. Trace amounts (0.2 % v/v) of nile red, a dye, was incorporated in the coating polymer solution to visually observe the homogeneity of the capsule coating and further evaluate their gastroresistance. The stability of the coatings of empty capsules was then evaluated in gradient pH media, simulating the GI environment at 37°C under agitation at 200 rpm. The dissolution media used were HCl solution at pH 1.2 (SGF) for 2h and phosphate buffer at pH 6.8 (SIF).

### EXAMPLE 11: In vivo anticoagulant activity

The anticoagulant activity of freeze-dried Fpx:Sq⁺ NPs filled into Eudragit^{®} L100 coated capsules of example 10 was evaluated *in vivo* after oral administration in male Sprague-Dawley rats. Different enteric-coated capsules were administrated orally to the rats (n=6 for each studied group) with help of a feeding needle (Harvard Apparatus, France): Fpx:Sq⁺ NPs-filled in enteric coated capsules at 10 mg/kg (eq. Fpx) with fasted and fed rats; Fpx-filled enteric-coated capsules at 10 mg/kg with fed rats. Blood samples were periodically taken from the jugular veins after anesthesia with isoflurane and the plasma concentration of active Fpx was estimated using an anti-FXa assay.

Are shown in figure 6, plasma Fpx concentration versus time profiles of rats after oral administration of Eudragit^{®} L100-coated capsules filled with: i) free-form of Fpx (10 mg/kg) **(--Δ--),** fasted rats; ii) freeze-dried NPs in fasted ( 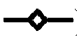 ) and fed rats ( 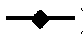 ); control groups ( 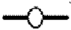 ) (n=6 for each studied group).

Figure 6 shows the plasma concentration-time profile of free Fpx as compared to encapsulated Fpx administered orally in enteric-coated capsules. As expected, a very low plasma Fpx concentration was observed in the rats orally treated with the Eudragit^{®} L100-coated capsules filled with the free form of Fpx (Cₘₐₓ of around 0.5 mg/L). The concentration-profile was similar to the control group. On the contrary, oral administration of enteric-coated capsules filled with Fpx-loaded NPs showed a maximum plasma concentration of 0.44 mg/L at 3h post-administration. The area under the plasma Fpx concentration versus time curve, AUC ₍₀₋₅ₕ₎ was 38.73 ± 19.01 mg.min/mL which corresponded to an absolute bioavailability of 9.01 ± 2.60 %. Compared to the oral absorption of nanoparticulate suspensions where the Tₘₐₓ was 1h (figure 2B), the maximum plasma concentration was obtained with enteric-coated capsules at 3h and the absolute bioavailability was increased by a factor of 30. It can be concluded that the use of enteric coatings delayed the Fpx absorption and improved the oral absorption of Fpx. Moreover, in order to determine the influence of food on oral absorption of Fpx, the NPs were administered orally to fasted rats (figure 6). The fasted rats orally treated with the Eudragit^{®} L100-coated capsules resulted in a maximum plasma concentration at 3h after treatment. The maximum plasma Fpx concentration decreased by 48 % with fasted rats. Thus, these data show that food enhance the oral absorption of Fpx and delay the peak plasma concentration. Accordingly, the Fpx:Sq⁺ nanoparticulate system effectively improves the oral delivery of Fpx as compared to the free Fpx.

## Claims

1. Nanoparticles comprising at least one negatively charged glycosaminoglycan-type macromolecule or a derivative thereof, non-covalently coupled to at least one molecule of a cationic hydrocarbon-based radical of squalene nature represented by the formula (I) which follows: 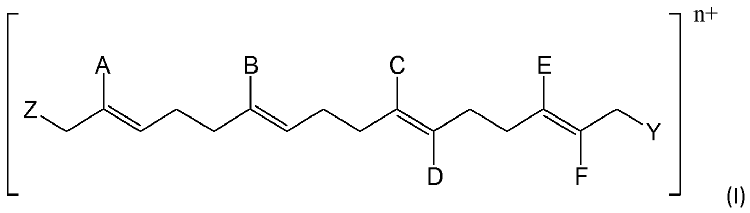 wherein:
- A, B, C, D, E and F are, independently ones from the others, a hydrogen atom or a methyl group,
- Z and Y, independently one from the other, represent a radical 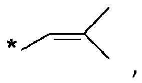 an ethyltrialkylammonium radical, an ethylguanidium radical or a 1-[ethylideneamino]guanidinium radical with the proviso that at least one of Z and Y is
different from 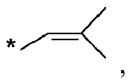 the symbol * showing the position of the attachment of the radical to the structure, and
- n is equal to 1 or 2.

2. Nanoparticles according to claim 1, wherein n is equal to 1.

3. Nanoparticles according to claim 1 or 2, wherein at least one Y represents one ethyltrialkylammonium radical and n is equal to 1.

4. Nanoparticles according to any one of claims 1 to 3, wherein Y and Z represent one ethyltrialkylammonium radical and n is equal to 2.

5. Nanoparticles according to any one of claims 1 to 4, wherein the negatively charged glycosaminoglycan-type macromolecule is fondaparinux.

6. Nanoparticles according to any one of claims 1 to 5, wherein the negatively charged glycosaminoglycan-type macromolecule is fondaparinux and the molecule of a cationic hydrocarbon-based radical of squalene nature is of formula 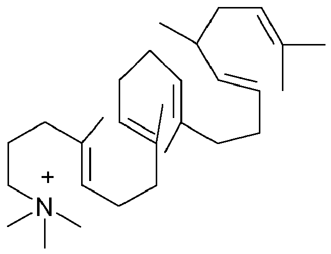 in a molar ratio fondaparinux/cationic hydrocarbon-based radical of squalene of 1:6.

7. Nanoparticles according to any one of claims 1 to 5, wherein the negatively charged glycosaminoglycan-type macromolecule is fondaparinux and the molecule of a cationic hydrocarbon-based radical of squalene nature is of formula 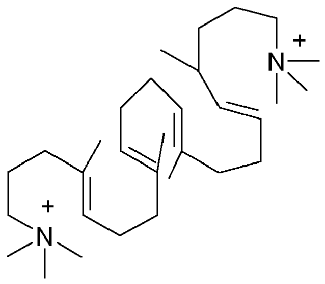 in a molar ratio fondaparinux/cationic hydrocarbon-based radical of squalene of 1:0.5.

8. Nanoparticles according to any one of claims 1 to 7, the mean size of these nanoparticles ranging from 50 to 800 nm, in particular from 75 to 500 nm, and more preferably from 100 nm to 200 nm.

9. Nanoparticles according to any one of claims 1 to 8, the zeta potential ranging from -75 mV to - 30mV, more preferably from -65mV to -35mV and still more preferably from -60 mV to -40mV.

10. Nanoparticles as defined in anyone of claims 1 to 9 for use as a medicine.

11. Nanoparticles as defined in anyone of claims 1 to 9 for use as anticoagulant agents for the treatment and/or prevention of thromboembolic diseases and cardiovascular diseases such as thrombophlebitis, venous thrombosis, phlebothrombosis, deep vein thrombosis, portal vein thrombosis, renal vein thrombosis, jugular vein thrombosis, Budd-Chiari syndrome, Paget-Schroetter disease, cerebral venous sinus thrombosis, pulmonary embolism, arterial thrombosis, stroke, myocardial infarction, hepatic artery thrombosis, arterial embolus.

12. Method for preparing the nanoparticles according to anyone of claims 1 to 9, comprising at least the steps consisting of:
- providing a solution of a cationic squalene compound of formula (I) as defined in claim 1 in a water-soluble organic solvent,
- pouring said solution in an aqueous phase containing at least one molecule of negatively charged GAG macromolecule, the concentration of compound of formula (I) as defined in claim 1 and of GAG macromolecule being adjusted to get an ionic bioconjugate which, under stirring, instantaneously forms the expected nanoparticles in suspension in said aqueous phase, and, where appropriate, the isolation of said nanoparticles.

13. Method according to claim 12 comprising further a step of freeze-drying.

14. Lyophilisate comprising at least nanoparticles as defined in anyone of claims 1 to 9.

15. Solid form dosage containing at least nanoparticles as defined in anyone of claims 1 to 9, optionally in the form of a lyophilisate.

16. Solid form dosage as defined in claim 15, which is a delayed release solid form dosage like for example enteric coated tablets or capsules and which surface coating provides the delayed release.

17. Pharmaceutical or dermatological composition comprising at least nanoparticles as defined in anyone of claims 1 to 9, optionally in the form of a lyophilisate, and/or capsules, in combination with at least one acceptable pharmaceutical vehicle.

18. Pharmaceutical or dermatological composition according to claim 17 comprising further a P-glycoprotein inhibitor.

19. Pharmaceutical or dermatological composition according to anyone of claims 17 or 18, wherein the P-glycoprotein inhibitor is chosen among verapamil, quinidine, amiodarone, reversin, matairesinol, sipholenol and cyclosporin.

20. Pharmaceutical or dermatological composition according to anyone of claims 17 to 19, said composition being orally administered.

21. Pharmaceutical or dermatological composition according to anyone of claims 17 to 19, said composition being systemically administered.
